# EUROPÄISCHE PATENTANMELDUNG

(11) **EP 3 088 033 A1**
(43) Veröffentlichungstag der Anmeldung: **02.11.2016**
(21) Anmeldenummer: 16000433.9
(22) Anmeldetag: 23.02.2016
(51) Int. Cl.: A61M 16/10, A61M 16/20, A61M 16/12, A61M 16/00, G01F 1/68

(54) **LACHGASMISCHER ZUR ERZEUGUNG EINES LACHGASGEMISCHES**

(30) Priorität: 27.04.2015 EP 15001240
(71) Anmelder: Baldus Medizintechnik GmbH, 56182 Urbar (DE)
(72) Erfinder: Baldus, Fabian, 56179 Vallendar (DE)
(74) Vertreter: Hannke, Christian

(57) **Zusammenfassung**

Die Erfindung betrifft einen Lachgasmischer (100) zur Erzeugung eines Lachgasgemisches, umfassend:
- zumindest eine Mischkammer (1), innerhalb derer ein Sauerstoffgas (11) und ein Lachgas (12) in jeweils vorgebbaren Volumina und/oder Molanteilen miteinander zu dem Lachgasgemisch (13) vermischt werden, wobei
- die Mischkammer zumindest einen Lachgasanschluss (120) und zumindest einen Sauerstoffgasanschluss (110) aufweist, so dass über entsprechend an diesen Anschlüssen montierte Gasleitungen (110A, 120A) sowohl das Sauerstoff- als auch das Lachgas in die Mischkammer eingeleitet wird,
- zumindest einen Konzentrationsregler (2), welcher dazu eingerichtet und dafür vorgesehen ist, ein Mischungsverhältnis zwischen dem in die Mischkammer eingeleiteten Sauerstoffgas und/oder dem in die Mischkammer eingeleiteten Lachgas vorgebbar einzustellen,
- zumindest einen Flussmengenregler (3), mittels dessen ein Volumenstrom der beiden Gase jeweils separat und/oder gemeinsam eingestellt werden kann,
wobei
zur Notflutung der Mischkammer mit dem Sauerstoffgas und/oder mit Umgebungsluft an der Mischkammer zumindest ein O₂-Notfallknopf (4) angebracht ist, wobei ein Ein- und Ausschaltknopf des Lachgasmischers verschieden von einem Regelelement ist, und der O2-Notfallknopf in einer Betätigungsrichtung (41) eindrückbar ist, wobei eine ebene Montagefläche (42) des O₂-Notfallknopfes, zu welcher die Betätigungsrichtung an zumindest einem Punkt in der Montagefläche senkrecht verläuft, frei von weiteren Regelelementen, ist, und sich daher in der gesamten Nachbarschaft entlang und in der Ebene der Montagefläche kein weiteres Regelelement befindet, und wobei

- die Montagefläche eine Außenfläche eines Mischkammergehäuses ist, an welcher der O2-Notfallknopf angeordnet ist, oder

- die Montagefläche eine Außenfläche einer Steuerungseinheit des Lachgasmischers ist, wobei eine solche Steuerungseinheit dann entfernt von dem Mischkammergehäuse angeordnet ist.

## Beschreibung

Die vorliegende Erfindung betrifft einen Lachgasmischer zur Erzeugung eines Lachgasgemisches gemäß dem Oberbegriff des Patentanspruches 1.

Der hier vorgestellte Lachgasmischer umfasst zumindest eine Mischkammer, innerhalb derer ein Sauerstoffgas und ein Lachgas in jeweils vorgebbaren Volumina und/oder Molanteilen miteinander zu dem Lachgasgemisch vermischt werden.

Dabei umfasst die Mischkammer zumindest einen Lachgasanschluss und zumindest einen Sauerstoffgasanschluss, so dass über entsprechend an diesen Anschlüssen montierte Gasleitungen sowohl das Sauerstoff- als auch das Lachgas in die Mischkammer eingeleitet werden.

Zudem umfasst der Lachgasmischer zumindest einen Konzentrationsregler, welcher dazu eingerichtet und dafür vorgesehen ist, ein Mischungsverhältnis zwischen dem in die Mischkammer eingeleiteten Sauerstoffgas und/oder dem in die Mischkammer eingeleiteten Lachgas vorgebbar einzustellen.

Des Weiteren umfasst der hier beschriebene Lachgasmischer zumindest einen Flussmengenregler, mittels dessen ein Volumenstrom der beiden Gase jeweils separat und/oder gemeinsam eingestellt werden kann.

Derartige Lachgasmischer zur Erzeugung eines Lachgasgemisches sind jedoch bereits aus dem Stand der Technik bekannt.

Bekannt sind dabei insbesondere auch solche Lachgasmischer, welche einen O₂-Notfallknopf umfassen. Mittels eines solchen O₂-Notfallknopfes kann Patienten im Fall einer durch das Lachgas erzielten Übersedierung oder im Fall von Kreislaufproblemen reiner Sauerstoff, insbesondere in die Mischkammer, eingeleitet werden. Denkbar ist jedoch auch, dass durch Betätigung des O₂-Notfallknopfs der Stickstoffanteil auf einen vorgegebenen Volumenprozentsatz, beispielsweise auf 5 Volumen-%, relativ zum Sauerstoffvolumen reduziert wird.

Der O₂-Notfallknopf ist nämlich ein solcher, welcher die Zugabe von Stickstoff vorzugsweise vollständig unterbindet und somit einen Stickstoffeinfluss des Lachgases in die Mischkammer verhindert. Der Patient kann insofern innerhalb kürzester Zeit mit reinem Sauerstoff versorgt werden. Es versteht sich von selbst, dass anstatt reinem Stickstoff auch beliebige andere Stickstoffverbindungen, beispielsweise Distickstoffmonoxid, in Frage kommen.

Allerdings weisen aus dem Stand der Technik bekannte Lachgasmischer nur solche O₂-Notfallknöpfe auf, welche versteckt beispielsweise hinter einem Konzentrations- und/oder einem Flussmengenregler angeordnet sind. Im Falle einer Übersedierung ist daher die Erfahrung gemacht worden, dass - obwohl ein O₂-Notfallknopf vorhanden ist - dieser oft übersehen wurde und aus diesem Grunde wertvolle Zeit verstrichen ist, bis der Benutzer den O₂-Notfallknopf auffinden konnte.

Ausgehend hiervon ist es daher eine Aufgabe der vorliegenden Erfindung, das oben genannte Problem zu lösen und insofern einen Lachgasmischer zur Erzeugung eines Lachgasgemisches anzubieten, bei dem es in besonders einfacher Art und Weise möglich ist, im Falle einer Übersedierung eines Patienten oder im Falle von Kreislaufproblemen des Patienten eine Notfall-Sauerstoffflutung in der Mischkammer des Lachgasmischers vorzunehmen.

Diese Aufgabe wird durch den Gegenstand des Patentanspruches 1 gelöst.

Um nun diese Aufgabe zu lösen und die oben genannten Probleme zu vermeiden, macht die vorliegende Erfindung daher unter anderem von der Idee Gebrauch, dass zur Notflutung der Mischkammer mit dem Sauerstoffgas an der Mischkammer zumindest ein O₂-Notallknopf angebracht ist, der in einer Betätigungsrichtung eindrückbar ist, wobei eine, vorzugsweise ebene, Montagefläche des O₂-Notfallknopfes zu welcher die Betätigungsrichtung an zumindest einem Punkt in der Montagefläche senkrecht verläuft, frei von weiteren Regelelementen ist.

Das heißt, dass sich daher in der gesamten Nachbarschaft in und entlang der Ebene der Montagefläche kein weiteres Regelelement befindet. Unabhängig vom Abstand eines Punktes in dieser Ebene und entlang deren gesamter Erstreckung relativ zu dem O₂-Notfallknopf ist daher vorzugsweise kein weiteres Regelelement angeordnet. Insbesondere kann die Montagefläche auch frei von Montage- und/oder Fixierelementen sein. Beispielsweise ist der O₂-Notfallknopf mittig auf der Montagefläche angeordnet.

Dies kann heißen, dass über die gesamte Erstreckung der Montagefläche an dieser keine Montage- und/oder Fixierelemente angeordnet sind. Vorzugsweise ragt daher bis auf den O₂-Notfallknopf entlang dieser Montagefläche (beispielsweise gebildet durch eine Außenfläche der Mischkammer) kein weiteres Element von der Mischkammer weg oder hinein. Auch kann diese Montagefläche frei von Bildschirmen oder sonstigen analogen oder digitalen Darstellungseinheiten zur Darstellung von z.B. Druck, Temperatur, Fließgeschwindigkeit sein.

Die Montagefläche ist daher beispielsweise diejenige Außenfläche eines Mischkammergehäuses, an welcher der O₂-Notfallknopf angeordnet ist. Die Montageflache kann jedoch auch eine diejenige Außenfläche einer Steuerungseinheit des Lachgasmischers sein, wobei eine solche Steuerungseinheit dann entfernt von dem eigentlichen Mischkammergehäuse angeordnet sein kann.

Dabei handelt es sich insbesondere bei einer ebenen Montagefläche um eine solche Montagefläche, welche frei von Spalten und Unterbrechungen ist. Insbesondere kann es sich jedoch bei einer ebenen Montagefläche daher um eine solche Fläche handeln, welche entweder krümmungsfrei ist oder aber zumindest eine Krümmung aufweist. Handelt es sich bei der Montagefläche um eine krümmungsfreie Fläche, stellt diese Montagefläche eine Montageebene dar. Auf einer solchen Montageebene verläuft daher die Betätigungsrichtung an jedem Punkt senkrecht.

Mit dem Begriff "Regelelemente" werden solche Elemente eines Lachgasmischers bezeichnet, welche zur Einstellung eines Mischungsverhältnisses und/oder zur Einstellung einer Flussmenge der Gase geeignet sind. Ein Ein- und Ausschaltknopf des Lachgasmischers ist daher verschieden von einem solchen Regelelement. Mit anderen Worten ist denkbar, dass neben dem O₂-Notfallknopf in der Montagefläche zusätzlich noch ein solcher Ein- und Ausschaltknopf angeordnet ist.

Insofern ist daher in besonders einfacher Art und Weise gewährleistet, dass der hier beschriebene O₂-Notfallknopf nicht durch andere Regelelemente für den Benutzer verdeckt ist oder optisch in den Hintergrund gerät, sondern insbesondere in einem Zentralbereich der Montagefläche angeordnet ist.

Gemäß zumindest einer Ausführungsform umfasst der Lachgasmischer zur Erzeugung eines Lachgasgemisches zumindest eine Mischkammer, innerhalb derer ein Sauerstoffgas und ein Lachgas in jeweils vorgebbaren Volumina und/oder Molanteilen miteinander zu dem Lachgasgemisch vermischt werden, wobei die Mischkammer zumindest einen Lachgasanschluss und zumindest einen Sauerstoffgasanschluss aufweist, so dass über entsprechend an diesen Anschlüssen montierte Gasleitungen sowohl das Sauerstoff- als auch das Lachgas in die Mischkammer eingeleitet wird.

Zudem umfasst der hier beschriebene Lachgasmischer zumindest einen Konzentrationsregler, welcher dazu eingerichtet und dafür vorgesehen ist, ein Mischungsverhältnis zwischen dem in die Mischkammer eingeleiteten Sauerstoffgas und/oder dem in die Mischkammer eingeleiteten Lachgas vorgebbar einzustellen, wobei zumindest ein Flussmengenregler, mittels dessen ein Volumenstrom der beiden Gase jeweils separat und/oder gemeinsam eingestellt werden kann, ebenso ein Teil des hier beschriebenen Lachgasmischers ist.

Erfindungsgemäß wird zur Notflutung der Mischkammer mit dem Sauerstoffgas und/oder mit Umgebungsluft an der Mischkammer zumindest ein O₂-Notfallknopf angebracht, der in einer Betätigungsrichtung eindrückbar ist, wobei eine, vorzugsweise ebene, Montagefläche des O₂-Notfallknopfes, zu welcher die Betätigungsrichtung an zumindest einem Punkt in der Montagefläche senkrecht verläuft, frei von Regelelementen ist.

Mit anderen Worten handelt es hierbei erstmals um einen solchen (insbesondere analoge) Lachgasmischer, bei welchem ein Alarm einer Alarmeinrichtung dem Benutzer mitteilt, wenn der Patient mit zu wenig Sauerstoff versorgt wird und infolgedessen der Benutzer oder aber auch vollautomatisch der O₂-Notfallknopf betätigt wird um eine Zufuhr von Lachgas zu dem Patienten vollständig zu unterbinden, sodass dieser, insbesondere nur, mit Sauerstoff versorgt wird. Zum besonders schnellen Auffinden kann der Notfallknopf mit Rot gekennzeichnet sein.

Insbesondere kann in diesem Zusammenhang somit vorstellbar sein, dass wenn der 02 Alarm ertönt, dies bedeutet, dass der Mischer mit zu wenig 02 versorgt wird. In diesem Falle fließt auch vorzugsweise kein Lachgas mehr (100% Lachgas können sogar tödlich sein, Lachgassperre bei 02-Ausfall) und die leere 02-Flasche muss getauscht werden oder eine Steckverbindung zu einem Gas-(O2)-Versorgungsnetz hat sich getrennt oder das Versorgungsnetz ist leer.

Es ist somit vorstellbar, dass wenn der Alarm ertönt der 02-Flush-Knopf nicht mehr gedrückt werden kann oder nicht mehr gedrückt zu werden braucht.

Grundsätzlich kann jedoch der hier beschriebene Lachgasmischer auch zur vorgebbaren Mischung anderer Gase zur Anwendung kommen. Beispielsweise kann dann das Lachgas durch ein anderes Gas ersetzt sein um dann mit dem Sauerstoff vermischt zu werden. Es ist allerdings ebenso möglich den Sauerstoff selbst durch ein anderes Gas zu ersetzen.

Zudem ist vorstellbar, dass der hier beschriebene Lachgasmischer nicht nur im Rahmen einer Zahnbehandlung sondern alternativ oder zusätzlich auch im Rahmen eines sonstigen Sedierungsprozesses, beispielsweise vor oder während einer Geburtsphase im Kreissaal, Anwendung findet. In diesem Kontext ist vorstellbar, dass im Rahmen einer gynäkologischen Behandlung der Lachgasmischer über ein Demandventil mit einer Mund-Nasenmaske verfügt.

Insbesondere sollte jedoch herausgestellt werden, dass der hier beschriebene Lachgasmischer über zumindest einen Eingangsdruckminderer verfügen kann, mittels dessen ein konstanter Systemdruck (einzelne Gasdrücke oder Gesamtdruck in der Mischkammer) gewährleistet werden kann.

Gemäß zumindest einer Ausführungsform umfasst die Mischkammer eine Vorkammer sowie eine Hauptkammer, wobei nur die Vorkammer die beiden Gasanschlüsse umfasst und die Hauptkammer über zumindest eine Fluidverbindung mit der Vorkammer verbunden ist, wobei ein Raumluftventil anstatt an einer Halterung für die Hauptkammer befestigt zu sein abseits dieser Halterung angeordnet ist.

Aus dem Stand der Technik sind nämlich nur solche Raumluftventile bekannt, welche an oder oberhalb der Hauptkammer, insbesondere an einer Halterung für die Hauptkammer befestigt sind. Handelt es sich bei der Hauptkammer um einen Gasbeutel, so ist oftmals im Stand der Technik das Raumluftventil an dieser oberhalb der Hauptkammer angeordneten Halterung angeordnet.

Dies führte jedoch oftmals zu einem ungewollten Einführen von durch das Raumluftventil von oben angesaugtem Blut, Speichel, etc.... Die über ein solches, dann über dem Gasbeutel angeordnetes Raumluftventil von oben angesaugten Verunreinigungen kontaminieren oftmals Innenflächen der Hauptkammer. Es wurde daher die Erfahrung gemacht, dass dies zwar eine platzsparende Anordnung des Raumluftventils ist, jedoch über das Raumluftventil beim Ansaugen der Luft über das Raumluftventil Staub, Blut, Speichel, Reinigungsmittel, etc. somit in die Hauptkammer laufen können.

Insofern wurde nunmehr erdacht, dass dieses Raumluftventil abseits einer derartigen Hauptkammer und insbesondere abseits einer derartigen Halterung für die Hauptkammer plaziert wird. Beispielsweise ist ein derartiges Raumluftventil an einer von der Halterung für die Hauptkammer verschiedenen Halterung angeordnet. Insbesondere kann eine Flächennormale der Ventilöffnung des Raumluftventils nach unten und im Wesentlichen parallel zur Hauptersteckungsrichtung der Hauptkammer verlaufen. Bei dem Raumluftventil kann es sich um ein Unterdruckventil handeln, welches sich automatisch beim Einatmen des Patienten dann öffnet, wenn die Hauptkammer von dem Patienten leer geatmet ist. Durch die Anordnung des Raumluftventils derart, dass dessen Öffnung nach unten, in Richtung des Bodens also, offen ist, werden somit keine Verunreinigungen angezogen. Das Raumluftventil ist somit vor Blut, Speichel, etc... geschützt. Das Raumluftventil dient insbesondere zum Versorgen des Patienten mit Umgebungsluft wenn ein Gasreservoir (z.B. ein Gasbeutel) leer ist.

Gemäß zumindest einer Ausführungsform sind zumindest der O₂-Notfallknopf, vorzugsweise jedoch auch der Konzentrationsregler und der Flussmengenregler, an der Vorkammer angeordnet. Insofern ist das System der Mischkammer unterteilt in ein Vorkammer- und ein Hauptkammersystem. Die Vorkammer ist dazu da, um ein in die Hauptkammer eingeleitetes Lachgas in seiner Zusammensetzung in Bezug auf den Sauerstoff- und den Lachgasanteil einzustellen, während aus der Hauptkammer das dann fertig zusammengestellte Lachgas in einen Entnahmeschlauch eingeleitet wird. Der Entnahmeschlauch kann dann mit einer entsprechenden Nasenmaske verbunden sein.

Gemäß zumindest einer Ausführungsform ist die Hauptkammer mit zumindest einem Gasbeutel gebildet. Insofern ist die Hauptkammer beispielsweise anstatt mit einem nicht deformierbaren Bauteil mit einem solchen Bauteil gebildet, welches zum einen nicht nur deformierbar ist, sondern sich darüber hinaus auch individuell in seiner Größe an die entsprechend benötigte Menge an Lachgas anpasst.

Gemäß zumindest einer Ausführungsform umfasst der hier beschriebene Lachgasmischer zumindest eine Mischersteuerungseinheit, wobei die Mischersteuerungseinheit zumindest eine digitale Steuerungseinheit umfasst, wobei die Steuerungseinheit derart ausgestaltet ist, dass diese den Konzentrationsregler und/oder den Flussmengenregler mittels der Vorgabe eines Benutzers steuert. Insbesondere kann die Mischersteuerungseinheit den Konzentrationsregler und/oder den Flussmengenregler auch regeln.

Mit anderen Worten sind also die hier beschriebene Mischersteuerungseinheit und insbesondere der hier beschriebene Lachgasmischer im Sinne der Anmeldung rein elektronisch gesteuert. Werden beispielsweise die digitale Steuerungseinheit und insbesondere ein Touchscreen dieser digitalen Steuerungseinheit (damit umfasst die Steuerungseinheit auch diesen Touchscreen) durch den Benutzer bedient, steuert also rein elektronisch diese Steuerungseinheit den Konzentrationsregler und/oder den Flussmengenregler mit zumindest einer Stelleinheit an.

Alternativ hierzu kann es sich bei der Mischersteuerungseinheit auch um eine solche handeln, welche eine analoge Steuerungseinheit umfasst. Eine derartige analoge Steuerungseinheit ist daher frei von einem Touchscreen und wird durch den Benutzer beispielsweise händisch, insbesondere nur händisch, gesteuert. Diese Mischersteuerungseinheit umfasst daher die beiden Regler selbst. Dazu umfassen der Konzentrationsregler und/oder der Flussmengenregler Eingriffselemente, mit denen der Benutzer beispielsweise händisch die Konzentration und die Flussmenge der einzelnen Gase an den Reglern steuern kann.

Insbesondere kann jedoch ein solcher analoger Lachgasmischer umfassend die analoge Steuerungseinheit ein akustisches Warnmittel umfassen. Ein solches Warnmittel kann jedoch auch in dem obig beschriebenen digitalen Lachgasmischer angeordnet sein.

Sollte nämlich der Fluss eines oder beider Gase unterbrochen werden, ertönt unmittelbar nach der Unterbrechung des jeweiligen Gasflusses ein insbesondere akustisches Warnsignal.

Insofern verfügt der hier beschriebene Lachgasmischer über ein derartiges akustisches Alarmpaket. Geht beispielsweise die Sauerstoffflasche leer, so kann ein Alarm ertönen. Wenn nämlich die Sauerstoffzufuhr unterbrochen ist, fließt somit auch kein Stickstoff mehr in die Mischkammer.

Insbesondere kann neben einer Stickstoffzufuhrunterbrechung auch eine Lachgaszufuhrunterbrechung realisiert sein.

Gemäß zumindest einer Ausführungsform umfasst die digitale Steuerungseinheit zumindest einen Betätigungsbildschirm, insbesondere einen Touchscreen, mittels dessen über eine händische Betätigung des Benutzers elektronisch, vorzugsweise nur elektronisch, der Konzentrationsregler und/oder der Flussmengenregler betätigbar sind.

Gemäß zumindest einer Ausführungsform ist die Steuerungseinheit dazu eingerichtet und dafür vorgesehen, dass während des Betriebs auf dem Behandlungsbildschirm eine Menüführung angezeigt wird, wobei über die Menüführung Behandlungsparameter, insbesondere Mengen- und/oder Flussangaben hinsichtlich des zugeführten Sauerstoff und/oder des Lachgases, individuell angezeigt werden.

Gemäß zumindest einer Ausführungsform ist die Steuerungseinheit dazu eingerichtet und dafür vorgesehen, dass nach dem Anwählen auf dem Betätigungsbildschirm die zuletzt behandelten Patienten angezeigt werden, wobei dies entweder mittels eines anonymisierten Kodierungssystems geschieht oder auch mit dem konkreten Namen bzw. der entsprechenden Patienten-ID durchgeführt wird.

Einer der Vorteile der Realisierung der Steuerungseinheit mittels des Betätigungsbildschirmes ist, dass der Benutzer sich nur auf die Anzeige des Betätigungsbildschirmes konzentrieren muss, um den Lachgasmischer in seiner Gesamtheit steuern zu können. Dabei kann der Betätigungsbildschirm zeitaktuelle Anzeigen der Lachgas- und/oder Sauerstoffgaskonzentration innerhalb des Lachgasgemisches sowie einzelne Flussmengen in Litern pro Minute oder auch eine Gesamtflussmenge des Gasgemisches umfassen. Derartige Zahlen- und Mengenangaben können daher durch Betätigung des Betätigungsbildschirmes besonders individuell, also über den Betätigungsbildschirm selbst, eingestellt werden.

Gemäß zumindest einer Ausführungsform umfasst der Betätigungsbildschirm zumindest ein Einstellfeld, auf dem der Benutzer händisch eine Konzentrationsänderung des Sauerstoffgases und/oder des Lachgases in vorgebbaren Änderungsschritten einstellen kann, wobei die Konzentrationsänderung vorzugsweise nur durch die Betätigung des Einstellfeldes einstellbar ist. Dies bietet den Vorteil, dass mittels beispielsweise eines einmaligen Drückens auf das Einstellfeld (= Drückknopf), d.h. mittels des Drückens eines einzigen Knopfes, eine Konzentrationsänderung beispielsweise des Sauerstoffgases aber auch alternativ oder zusätzlich des Lachgases eingestellt werden kann. Standardmäßig kann daher der Lachgasmischer so eingestellt sein, dass der Betätigungsbildschirm darauf eingestellt ist, den Sauerstoff zu einer vorgegebenen Menge von Lachgas in 5 Prozentschritten hinzuzufügen.

Drückt nun der Benutzer auf dieses Einstellfeld (welches beispielsweise mit "Konzentrationsänderung: 1%/5%" benannt ist), so wird das Lachgas in ein Prozentschritten erhöht bzw. reduziert. Eine Umstellung ist daher jederzeit möglich, z.B. wenn man ein Kind sedieren möchte und das Lachgas fein titrieren muss.

Daneben ist jedoch auch denkbar, dass die Patienten-ID in der digitalen Steuerungseinheit hinterlegt ist oder abgespeichert wird. Beispielsweise öffnet sich vor der Sedierung ein Tippfeld des Betätigungsbildschirmes mit Buchstaben und Ziffern, um einen Patienten genau mit Namen oder Patientennummer anlegen zu können, was entscheidende Vorteile für die Dokumentation der behandelnden Person mit sich bringt.

Zudem kann durch die Steuerungseinheit auf dem Behandlungsbildschirm ein Menüpunkt "letzte Patienten aufrufen" angezeigt sein. Dies kann von dem Benutzer auch angewählt werden, so dass nach dem Anwählen auf dem Betätigungsbildschirm die zuletzt behandelten Patienten angezeigt werden. Dies kann entweder mittels eines anonymisierten Kodierungssystems geschehen oder auch mit dem konkreten Namen bzw. der entsprechenden Patienten-ID.

Es können auch die Behandlungszeit, der Beginn der Sedierung, Datum und maximaler Lachgaswert angezeigt werden. Außerdem besteht die Möglichkeit, den Patienteneintrag zu löschen, wobei auf dem Betätigungsbildschirm angeordnete und erscheinende Pfeile zum Weiterklicken auf den nächsten Patienten dienen können.

Zudem können über den hier beschriebenen Betätigungsbildschirm neben einer Konzentrationsänderung noch weitere Einstellungen vorgenommen werden.

Diese weiteren Einstellungen können Uhrzeit/Datum umfassen. Dabei können nämlich Uhrzeit und Datum beliebig angepasst werden. Auch können eine Lautstärke des Alarms im Falle einer Zufuhrunterbrechung eines der Gase und entsprechende Tastentöne besonders einfach eingestellt und reguliert werden. Auch können entsprechende Tastentöne vollständig ausgestellt werden.

Zudem bietet der hier beschriebene Betätigungsbildschirm die Möglichkeit, eine Servicekultur für den Lachgasmischer zu etablieren. Möglich ist es nämlich, den letzten und den nächsten Servicetermin angezeigt zu bekommen. Außerdem ist es möglich, die Hardware/Software anhand ihrer Seriennummer des Gerätes zu identifizieren, was wiederum auf dem Betätigungsbildschirm angezeigt werden kann.

Standardmäßig kann daher auch der Lachgasmischer so ausgeliefert werden, dass auf dem Betätigungsbildschirm prozentual angezeigt wird, wie viel Volumenprozent Lachgas in die Hauptkammer, fließen. Drückt nun der Anwender auf "Anzeige O₂/N₂O", so wird die prozentuale Sauerstoffkonzentration auf dem Sedierungsdisplay angezeigt. Eine Umstellung ist jederzeit möglich.

Zudem kann die hier beschriebene digitale Steuerungseinheit einen Fehlerspeicher, der eine vorgegebene Anzahl von Fehlermeldungen, beispielsweise von 20 Fehlermeldungen des Gerätes anzeigt und speichert, vorhanden sein.

Es besteht zudem die Möglichkeit, den Speicher zu löschen. Bei der Wartung kann insbesondere über eine Master-SD-Karte/Dongl für Serviceeinsätze eine vorgegebene Anzahl von Fehlern, vorzugsweise alle Fehler, aufgeführt werden. Die Fehler sind beispielsweise in einem Administratormenü immer nachzulesen und zu exportieren. Insbesondere kann zudem der Behandler mit einem USB Stick und/oder einem SB-Port Protokolle z.B. mit einer Patientennummer speichern. Zudem kann der Behandler die Behandlungsdauer, die Konzentration etc. dokumentieren.

Gemäß zumindest einer Ausführungsform umfasst der Lachgasmischer zumindest einen USB-Port und/oder einen SD-Kartenanschluss, sodass für Serviceeinsätze eine vorgegebene Anzahl von Fehlern, vorzugsweise alle Fehler, ausführbar sind, insbesondere wobei
- die Fehler in einem Administratormenü immer nachzulesen und zu exportieren sind, und/oder
- der Behandler mit einem USB Stick und/oder einem SB-Port Protokolle z.B. mit einer Patientennummer speichern kann, und/oder
- der Behandler die Behandlungsdauer, die Konzentration etc. dokumentieren kann.

Zudem ist es möglich, dass die hier beschriebene digitale Steuerungseinheit internetfähig ist. Beispielsweise weist die hier beschriebene digitale Steuerungseinheit ein LAN- oder WLAN-Modul auf, mit dem sich die digitale Steuerungseinheit gemäß zumindest einem standardisierten Internetprotokoll mit einer anderen digitalen Steuerungseinheit und/oder mit einem entfernt platzierten Administrator verknüpfen kann. Beispielsweise kann ein derartiger Administrator daher über die LAN- oder WLAN-Verbindung sich in die digitale Steuerungseinheit von extern einloggen, um somit einen Remote-Support gewährleisten zu können. Auch ist es möglich, dass mittels einer derartigen Steuereinheit ein Administrator einen Boot oder Reboot oder eine Ferndiagnose oder auch Ferneinstellungen an der digitalen Steuerungseinheit vornehmen kann.

Gemäß zumindest einer Ausführungsform umfasst der Lachgasmischer einen Fernbedienungsanschluss für eine Remotecontrol, wobei somit die Steuerungseinheit einen Kabel- und/oder IR- und/oder Bluetoothanschluss umfasst und/oder über Funk betreibbar ist.

Beispielsweise können über eine Smartphone oder Tablet Applikation über eine Touchscreeneingabe dieselben Funktionen bedient werden, wie über das Display des Lachgasmischers (die Lachgaskonzentration und den Gesamtflow erhöhen reduzieren, O2-Flush geben, ausleiten, Behandlungszeit erkennen etc.).

Zudem kann der hier beschriebene Lachgasmischer neben dem O₂-Notfallknopf eine Lachgassperre, ein Limitierungselement der Lachgaskonzentration und unterschiedliche Anschlussgrößen über Lachgas und Sauerstoff aufweisen.

Gemäß zumindest einer Ausführungsform ist an der Mischkammer zumindest ein Drucksensor angeordnet, welcher zumindest einen der Gasdrücke misst, wobei über eine mit dem Drucksensor innerhalb eines Regelkreises verbauten Regeleinheit die einzelnen Gasdrücke während der Behandlung oder auch vorher einstellbar und/oder ablesbar sind. Bei den Gasdrücken kann es sich insbesondere um diejenigen Drücke handeln, welche in Versorgungsflaschen des Lachgasmischers vorherrschen. Zum Beispiel kann über die Messung des jeweiligen in einer oder in mehreren der Gasflaschen auf einen Füllstand dieser Gasflaschen rückgeschlossen werden. Dies ermöglicht es in besonders einfacher Art und Weise auf eine Restbetriebszeit rückzuschließen.

Es wäre auch möglich durch die Kenntnis des Flaschendrucks Rückschlüsse auf die mögliche Restlaufzeit durch den Rückschluss auf das zur Zeit der Behandlung verabreichte Gas/ Minute zu berechnen. (Meldung: z.B. "Bei gleichbleibendem Atemminutenvolumen/Flow noch ca. X Minuten zur Verfügung" oder "Achtung in weniger als 30 Minuten ist das Gas leer").

Des Weiteren kann neben dem Drucksensor insbesondere auch durch Einbeziehung zumindest eines der Flowmeter in den Regelkreis über eine Recheneinheit voraus berechnet werden wie lange die Abgabe eines entsprechenden Gases aus der jeweiligen Gasflasche noch unter eine vorgebbaren Druck aufrechterhalten werden kann.
Gemäß zumindest einer Ausführungsform umfasst der Lachgasmischer neben dem O₂-Notfallknopf zudem einen Lachgasausleitknopf, über den eine Ausleitung des Lachgases, beispielweise aus der Mischkammer, sofort über die Dauer eines von einem Benutzer individuell einstellbaren Zeitraums unternehmbar und/oder über den Lachgasausleitknopf eine Zufuhr von Lachgas in die Mischkammer für die Dauer dieses Zeitraums sofort unterbrechbar ist, und so nur noch Sauerstoff in die Mischkammer zugeführt wird. Dazu kann der Lachgasausleitknopf in mechanischer und/oder elektrischer Wirkverbindung mit einem Entlüftungsventil der Mischkammer stehen, über welches das Lachgas aus der Mischkammer heraus entlassen werden kann.

### Denkbar ist in diesen Zusammenhang folgendes Betriebsszenario:

Über den Lachgasausleitknopf kann die Sedierung (Lachgas) sofort aus der Mischkammer ausgeleitet werden; es läuft dann ein Countdown ab. In dieser Zeit fließen nur noch 100 % 02 und es fließt kein Lachgas mehr. Nach den z.B. 5 Min. reinem Sauerstoff ist die Wirkung des Lachgases verflogen, da Lachgas nicht verstoffwechselt wird und schnell abgeatmet wird. Der Benutzer kann die Ausleitungszeit individuell einstellen.

Statt der Anordnung eines separaten Knopfes für die obig genannten Zwecke kann obig beschriebene sofortige Ausleitung oder Unterbrechung der Zufuhr des Lachgases auch der obig beschriebene O₂-Notfallknopf übernehmen. Dazu ist vorstellbar, dass über ein Drücken des O₂-Notfallknopfes in einer ersten Einstellung, vorzugsweise nur, Sauerstoff in die Mischkammer zugeleitet wird und in einer zweiten Stellung über den O₂-Notfallknopf zudem oder stattdessen aus der Mischkammer noch sich darin befindliches restliches Lachgas ausgeleitet wird. In der zweiten Stellung wird daher eine Entlüftung der Mischkammer vorgenommen.

Gemäß zumindest einer Ausführungsform umfasst der Lachgasausleitknopf ein Zeitsteuerelement oder wird von einem solchen gesteuert, sodass sich nach Ablauf einer an dem Zeitsteuerelement eingestellten Zeitdauer der Lachgasausleitkopf selbsttätig wieder in eine Nullstellung zurückstellt. Die Nullstellung ist daher diejenige Einstellung des Lachgasausleitknopfes, in welcher dieser in einer Aus-Stellung bewegt ist.

Gemäß zumindest einer Ausführungsform ist an oder in zumindest einer der Gasleitungen und/oder unmittelbar an der Mischkammer zumindest ein Flussmengensensor zur Messung einer Fließgeschwindigkeit und/oder eines Volumenstroms des jeweils in den Gasleitungen geleiteten Gases und/oder zur Messung eines Vakuumdurchflusses einer Absaugung angeordnet. Beispielsweise können diese Aufgaben durch einen gemeinsamen und oder jeweils unterschiedliche Sensoren, je nach Aufgabentrennung, durchgeführt werden.

Wenn der Gasfluss den Sensor passiert, wird Wärme von dem Sensor zu einem Medium, beispielsweise im Medium, geführt. Bei Zunahme des Flusses nimmt auch die Menge der Wärme, welche übertragen wird, zu. Wenn die Wärmeübertragung bekannt ist, kann die Durchflussmenge durch den Betrag des Spannungsausgleichs bestimmt werden, der benötigt wird, um einen konstanten Temperaturunterschied aufrechtzuerhalten.

Insbesondere kann die gesamte Vorrichtung frei von Messpropellern zur Messung eines Gasvolumenstroms sein. Stattdessen kann daher ausschließlich der obig beschriebene Flussmengensensor zur Messung des Gasvolumenstroms dienen. Im Gegensatz zu den "Propellern" arbeitet der Sensor im Wesentlichen Druck und beispielsweise sogar auch Temperatur unabhängig. Auch ist die Reaktionszeit kürzer, da die Massenträgheit der Propeller zu einem verzögerten An- und Ablaufen führt.

Gemäß zumindest einer Ausführungsform ermittelt dieser Flussmengensensor die Fließgeschwindigkeit und/oder den Volumenstrom des jeweiligen Gases, vorzugsweise ausschließlich, basierend auf einer Wärmetransportmessung des Gases.

### Eine Absaugregulation direkt über die Mischkammer kann zudem wie folgt aussehen:

Das Lachgas-Sauerstoffgemisch muss zum Schutze des Personals abgesaugt werden (Vorgabe beispielsweise Vakuum-Absaugleistung 45L/Min.).

Bei dem digitalen Mischer kann ein zusätzlicher Flowsensor zum Messen des Vakuumdurchflusses der Absaugung integriert werden. Es ist noch eine weitere Messkammer im Mischer vorhanden und es handelt sich um ein Thermo- oder Ultraschall-Messverfahren.

Die Software verarbeitet den Input der Flowsensoren und auf dem Touchscreen-Display wird angezeigt wie stark der momentane Durchfluss/ die momentane Absaugleistung ist. Es soll auch möglich sein auf dem Touchscreen angezeigt zu bekommen wieviel l/min in Echtzeit abgesaugt werden. Außerdem soll es möglich sein, dass die Absaugleistung via Touchscreen-Einstellung verändert werden kann.

Eine Minimierung der Absaugleistung erfolgt durch ein kleiner werdendes Lumen eines Regelventils oder Magnetventils, eine Erhöhung der Absaugleistung über ein größer werdendes Lumen.

Im Display wird angezeigt, ob der Durchfluss in Ordnung ist (z.B. bei 45 UMin., grün hinterlegt). Ist der Durchfluss zu stark oder zu schwach, so ist es rot hinterlegt. Die Software erkennt automatisch, wenn der Durchfluss zu gering ist. Dann veranlasst sie, dass das Regelventil geöffnet wird und wenn der Durchfluss immer noch zu gering ist, kann das Regelventil auch ganz aufgestellt werden, so dass keine Minimierung des Durchflusses durch das Regelventil erfolgt. Ist der Durchfluss zu stark, erkennt die Software dies und schließt das Regelventil etwas, sodass wir bei ca. 45L/Min. sind. Außerdem kann man über das Touchscreen Display auch das Regelventil komplett schließen um den Flow zu stoppen.

Das Regelventil vom Vakuum öffnet sich erst, wenn Lachgas fließt, welches die Raumluft kontaminiert, erst dann läuft also die Absaugung. Somit kann der Patient ohne Widerstand durch die Absaugung reinen Sauerstoff atmen, weil dann die Absaugung geschlossen ist. Erst beim Abschalten des Lachgases mit einer Verzögerung von z.B. 30 Sekunden, schließt sich wieder das Regelventil und die Absaugung ist geschlossen. Der Nachlauf von ca. 30 Sekunden ist wichtig, weil sich noch Lachgas in der Lunge des Patienten befindet, welches noch abgeatmet werden muss.

Gemäß zumindest einer Ausführungsform umfasst der Lachgasmischer Gasventile zur Steuerung und/oder Durchleitung des Sauerstoffs und des Lachgases, wobei die Gasventile als Steuerungsmittel, anstatt Gummimembranen, jeweils einen Steuerkolben vorsehen.

Insofern kann der obig beschriebene Lachgasmischer frei von Membranen, insbesondere Gummimembranen, sein. Gummimembranen werden im Rahmen einer Ventilsteuerung oftmals wegen Ihres günstigen Materialpreises verwendet. Es wurde jedoch erkannt, dass bei längerer Benutzungsdauer und/oder hohen Druckunterschieden solche Gummimembranen oft rissig werden und dann nach kurzer Zeit sogar undicht werden können. Statt der Verwendung von Gummimembranen wurde nun erdacht entsprechende Ventilkolben zu verwenden. Dabei haben die Ventilkolben die gleiche Schaltfunktion wie die obig beschriebenen Gummimembranen. Dies unter anderem nämlich deshalb, da die Kolben innerhalb von Kolbengehäusen geführt sind, und dabei die Kolben im Wesentlichen zwei Schaltzustände einnehmen können. In einem ersten Schaltzustand ist daher eine Fluidverbindung zwischen einen Ventileingang und einem Ventilausgang zumindest teilweise frei für das Gasfluid, wobei in einem zweiten Schaltzustand der Kolben einen Durchgang für das Fluid zwischen einem Ventilausgang und einem Ventileingang zumindest teilweise versperrt.

Gemäß zumindest einer Ausführungsform ist der hier beschriebene Lachgasmischer frei von einem An- und Ausschaltknopf. Insofern kann vermieden werden, dass der Benutzer sich beispielsweise im Falle eines Notfalls nicht im Klaren ist, dass der Lachgasmischer über einen derartigen Ein- und Ausschaltknopf auszuschalten ist. Der hier beschriebene Lachgasmischer kann daher frei von einem derartigen An- und Ausschaltknopf und kann daher im Betrieb einfach durch Ein- und Ausstecken zu einer Steckdose an das Stromnetz angeschlossen oder von diesem getrennt werden.

Alternativ jedoch kann der Lachgasmischer trotzdem einen An- und Ausschaltknopf dann umfassen, wenn dieser An- und Ausschaltknopf derart ausgestaltet ist, dass dieser nur durch beständiges Drücken über einen vorgegebenen Zeitraum, beispielsweise zumindest zwei Sekunden, gedrückt bleibt, damit der Behandler nicht aus Versehen das Gerät ausschaltet, wenn er den O2-Notfallknopf betätigen will. Vorteilhaft befindet sich der Ein/Aus-Schalter nicht auf dem Touchscreen.

Insbesondere kann mittels des hier beschriebenen Lachgasmischers zudem ein Gesamtflow erhöht werden. Beispielsweise ermöglicht nämlich der hier beschriebene Lachgasmischer einen Gesamtfluss von 20l/min. oder höher, so dass die Hauptkammer, also beispielsweise der Atembeutel, stets ausreichend mit dem Lachgas gefüllt ist und die Sedierung wirken kann.

Ist nämlich ein derartiger Gesamtflow zu gering gewählt, beispielsweise im Bereich von nur 8 - 10 I/min., atmet häufig der Patient die Hauptkammer leer, weil ein aufgeregter Patient schneller und mehr I/min atmet und es öffnet sich somit das Raumventil, so dass die Umgebungsluft angesaugt wird und die Sedierung somit nicht wirken kann. Insbesondere hat sich nämlich ergeben, dass ein Volumen der Hauptkammer im Bereich von <10l auch deshalb oftmals zu gering gewählt ist, weil im Falle von aufgeregten Patienten ein eine sehr schnelle Atemfrequenz vorliegt

Wie bereits obig erwähnt, ist zudem eine Ablesbarkeit der Lachgaskonzentration im Vergleich zu dem Stand der Technik entscheidend verbessert. Im Vergleich zu bereits bekannten Lachgasmischern wird nämlich hier unter anderem auch vorgeschlagen, dass eine prozentuelle Lachgaskonzentration an der von dem Patienten in seiner Gesamtheit eingeatmeten Gasmenge angezeigt wird anstatt dass lediglich eine prozentuelle Sauerstoffgaskonzentration angezeigt wird. Somit muss die behandelnde Person nicht mehr umrechnen und es werden auch Fehleinstellungen vermieden.

Aus Sicherheitsgründen beginnt daher die hier vorgeschlagene Konzentrationsskala an Lachgas bei 0% Lachgas und endet bei 50% bzw. 60% bzw. 70% Lachgas (je nach Variante). Auch kann ein Gesamtflowanschlag und/oder ein separater Lachgaskonzentrationsregleranschlag an dem Lachgasmischer angeordnet sein, welcher Risiken bei der Bedienung des Gasflusses minimiert. Mittels eines derartigen Anschlages ist es nämlich vermieden, dass zu viel Gas fließt. Insbesondere kann der Gesamtflowanschlag es verhindern, dass mehr als z. B. 20 Liter pro Minute gemischtes Gas fließen.

Gemäß zumindest einer Ausführungsform umfasst der Lachgasmischer eine Sprachsteuerungsvorrichtung, wobei diese dazu eingerichtet und dafür vorgesehen ist, vorzugsweise alle, Funktionen über eine akustische Detektion einer Behandlerstimme zu steuern. Ein Sprachsignal, welches von der Sprachsteuerungsvorrichtung detektiert wird kann beispielsweise diverse Sprachkommandos wie "Gesamtflow 10 L/Min", Absaugung schließen", etc. betreffen.

Im Folgenden wird der hier beschriebene Lachgasmischer anhand von Ausführungsbeispielen und den dazugehörigen Figuren näher beschrieben:
Die Figuren 1A, 1B, 1C zeigen in einer schematischen Seitenansicht ein Ausführungsbeispiel eines hier beschriebenen analogen Lachgasmischers.

Die Figuren 2a und 2b zeigen in schematisch perspektivischen Ansichten ein Ausführungsbeispiel eines hier beschriebenen digitalen Lachgasmischers.

In den Figuren sind gleiche oder gleichwirkende Bestandteile jeweils mit den gleichen Bezugszeichen versehen. Die hier dargestellten Elemente sind nicht als maßstabsgerecht anzusehen, vielmehr können einzelne Elemente zum besseren Verständnis übertrieben groß dargestellt sein.

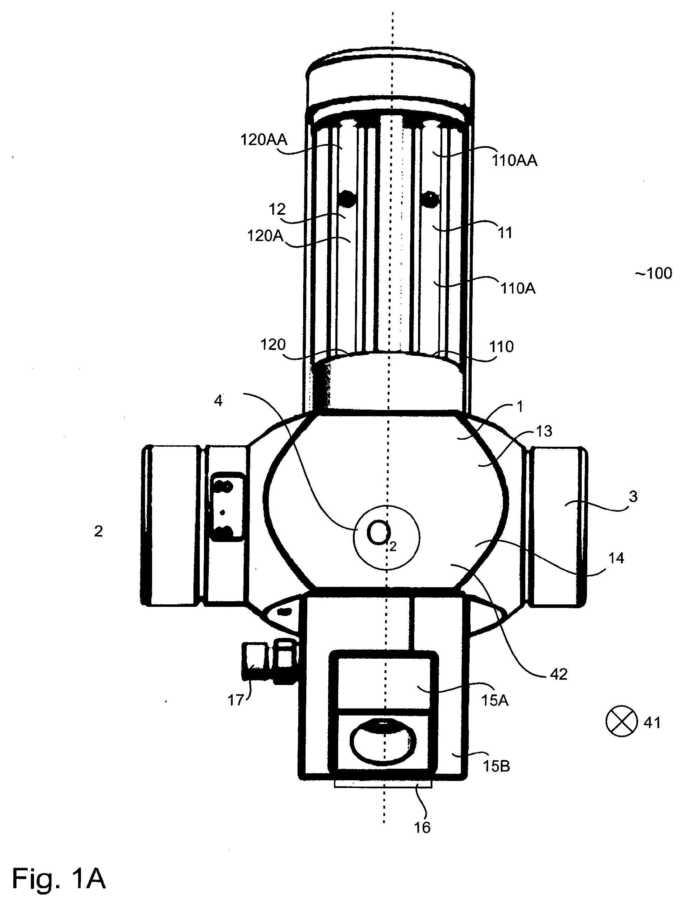

In der Figur 1A ist ein Ausführungsbeispiel eines hier beschriebenen analogen Lachgasmischers 100 gezeigt.

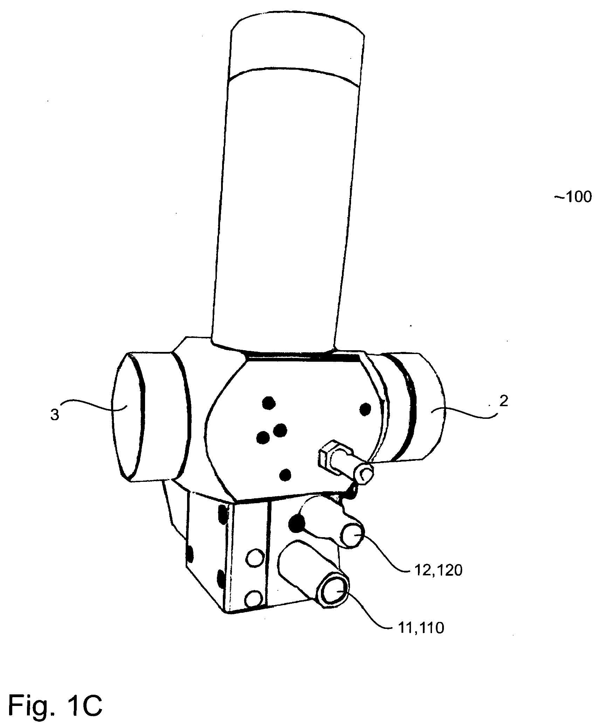

Aus dieser Figur ist besonders einfach erkennbar, dass der hier beschriebene Lachgasmischer 100 zur Erzeugung eines Lachgasgemisches zumindest eine Mischkammer 1 umfasst, innerhalb derer ein Sauerstoffgas 11 und ein Lachgas 12 in jeweils vorgebbaren Volumina und/oder Molanteilen miteinander zu dem Lachgasgemisch 13 vermischt werden, wobei die Mischkammer 1 einen Lachgasanschluss 120 (siehe Figur 1C) und einen Sauerstoffgasanschluss 110 (siehe Figur 1C) aufweist, so dass über entsprechend an diesen Anschlüssen montierte Gasleitungen 110A, 120A sowohl das Sauerstoff- als auch das Lachgas in die Mischkammer 1 eingeleitet werden. Insofern zeigt die Figur 1C eine perspektivische Rückansicht der in der Figur 1A gezeigten Vorderansicht des Lachgasmischers.

Insbesondere sind die Gasleitungen 110A (Sauerstoff), 120A (Stickstoff) derart ausgestaltet, dass diese jeweilige Flowmeter 110AA und 120AA umfassen, anhand derer die jeweilige Flussgeschwindigkeit der Gase über eine druckverschiebliche Flowkugel anhand der jeweiligen Messskalen abgelesen werden kann.

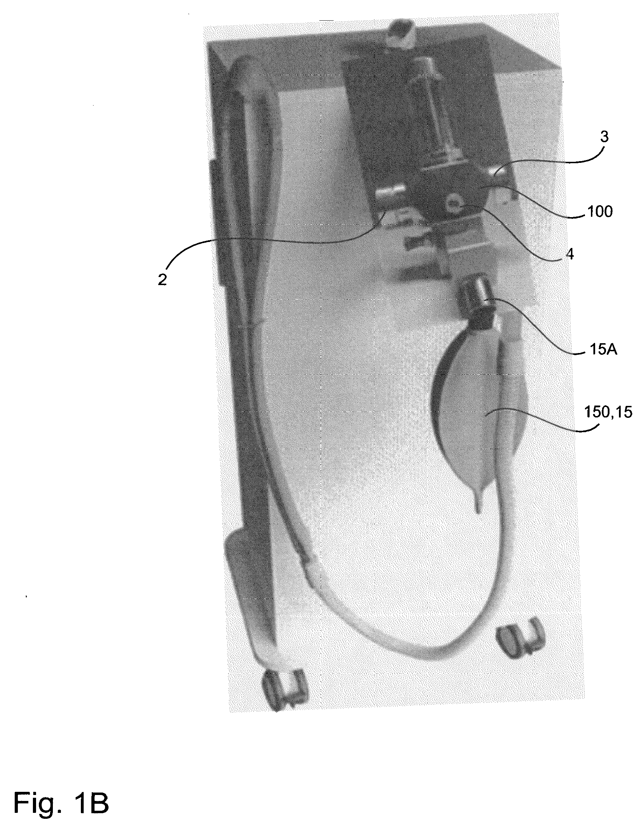

Zudem ist aus der Figur 1 ein Konzentrationsregler 2 erkennbar, mittels dessen ein Mischungsverhältnis zwischen dem in die Mischkammer 1 eingeleiteten Sauerstoffgas 11 und/oder dem in die Mischkammer 1 eingeleiteten Lachgas 12 vorgebbar eingestellt werden kann, wobei ebenso ein Flussmengenregler 3 erkennbar ist, mittels dessen ein Volumenstrom der beiden Gase 11, 12 separat und/oder gemeinsam eingestellt werden kann.

Besonders prominent aus der Figur 1 erkennbar ist der stets vorhandene und erfindungsgemäße O₂-Notfallknopf 4, welcher zur Notflutung der Mischkammer 1 mit dem Sauerstoffgas 11 und/oder mit Umgebungsluft dient.

Wird nun der O₂-Notfallknop 4 gedrückt, gelangt in die Mischkammer 1 ausschließlich nur noch das Sauerstoffgas 11. Eine Zufuhr des Lachgases 12 ist daher vollständig verhindert. Dazu ist der O₂-Notfallknop 4 an einem Mischkammergehäuse 1A der Mischkammer 1 angeordnet und in einer Betätigungsrichtung 41 eindrückbar, wobei das Mischkammergehäuse der Mischkammer 1 eine Montageebene 42 umfasst.

Der O₂-Notfallknop 4 ist daher an dieser Montagefläche 42 mit der Mischkammer 1 wirkverbunden und auch in Richtung der Montagefläche 42 in der Betätigungsrichtung 41 eindrückbar.

Entscheidend ist nun, festzustellen, dass die Montagefläche 42 frei von weiteren Regelelementen ist. Dies heißt insbesondere, dass der Konzentrationsregler 2 und der Flussmengenregler 3 nicht an der Montagefläche 42 des Mischkammergehäuses der Mischkammer 1 angeordnet sind, sondern an davon verschiedenen Seiten des Mischkammergehäuses 1A der Mischkammer 1 angeordnet sind. Dies hat unmittelbar zur Folge, dass die Anordnungsseite des O₂-Notfallknopfes 4 und die Anordnungsseiten des Konzentrationslegers 2 und/oder des Flussmengenreglers 3 an dem Mischkammergehäuse 1A der Mischkammer 1 voneinander verschieden sind. Insofern ist garantiert, dass in einem Notfall der Benutzer den O₂-Notfallknopf 4 sofort finden kann und dieser weder durch den Konzentrationsregler 2 noch durch den Flussmengenregler 3 oder weitere davon verschiedene Regelelemente verdeckt ist.

Aus der Figur 1B ist der in der Figur 1A beschriebene Lachgasmischer 100 in einer perspektivischen Ansicht erkennbar, wobei im Unterschied zu der Figur 1A nun besser erkennbar ist, dass die Mischkammer 1 mit einer Vorkammer 14 sowie mit einer Hauptkammer 15 gebildet ist. Die Hauptkammer 15 bildet einen Gasbeutel 150 aus. Der Gasbeutel 150, d.h. die Hauptkammer 15, ist mittels einer Fluidverbindung mit der Vorkammer 14 verbunden.

Erkennbar ist auch ein Raumluftventil 16, welches anstatt an der Halterung 15A (Bag T) für die Hauptkammer 15 befestigt zu sein abseits der Hauptkammer 15 angeordnet ist, um zu vermeiden, dass durch den Patienten während der Behandlung, jedoch insbesondere auch beim Einatmen Speichel, Blut, etc.in das Bag T in den Beutel (z. B. Spritzwasser etc.) gelangen. Insbesondere kann aus der Figur 1B erkannt werden, dass das Raumluftventil 16 an einer weiteren Halterung 15B angeordnet ist und in Richtung der Hauptkammer 15, beispielsweise auch in Richtung einer Längsachse L des Lachgasmischers 100, schließ- und öffenbar ist. Wird nun die Hauptkammer 15 durch den Patienten leergeatmet, öffnet während eines Einatmens des Patienten nach dem, insbesondere vollständigen, Entleeren der Hauptkammer 15 automatisch das Raumluftventil 16, so dass eine Fluidleitung, welche zwischen einem Nasenmaskenanschluss 17 des Lachgasmischers 1 und der Hauptkammer 15 geführt ist mit Umgebungsluft gefüllt wird. Der Patient ist somit stets ausreichend mit Sauerstoff versorgt.

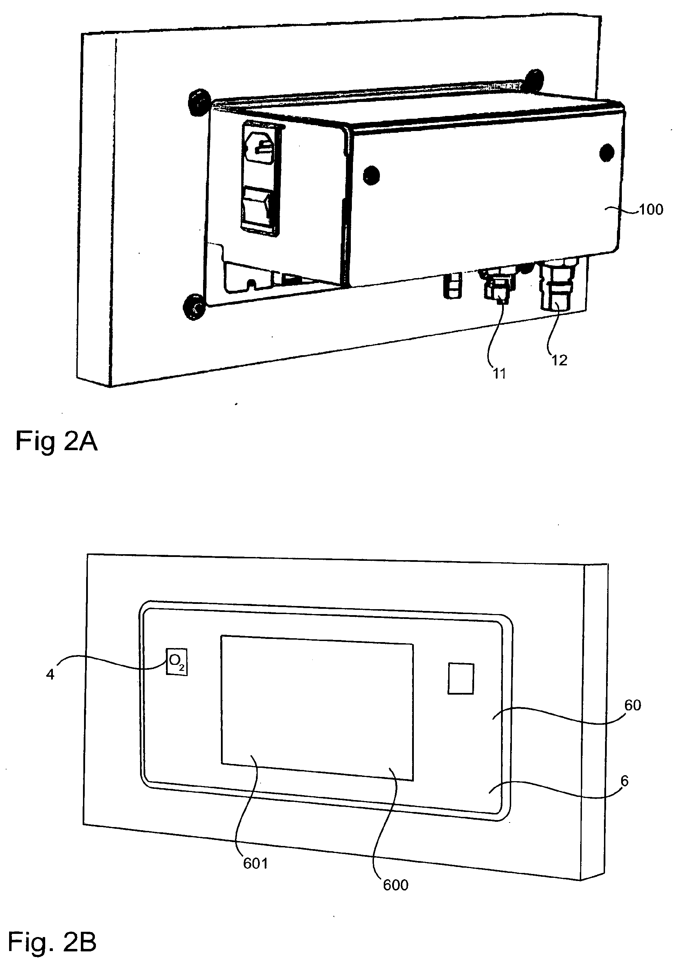

In den Figuren 2A und 2B ist in einer schematisch perspektivischen Ansicht ein Ausführungsbeispiel eines hier beschriebenen digitalen Lachgasmischers 100 gezeigt.

Wie aus der perspektivischen Rückansicht gemäß der Figur 2A erkannt werden kann, ist der beispielsweise wie in den Figuren 1A, 1B gezeigte Lachgasmischer 100 in einem Gehäuse eingebracht, wobei die verschiedenen Anschlüsse aus dem Gehäuse herausragen, um mit den entsprechenden Gasleitungen verbunden zu werden.

In der Figur 2B ist eine Vorderansicht erkennbar, in welcher eine Mischungssteuerungseinheit 6 dargestellt ist, welche eine digitale Steuerungseinheit 60 umfasst. Dabei ist die digitale Steuerungseinheit 60 derart ausgestaltet, dass diese den Konzentrationsregler 2 und/oder den Flussmengenregler 3 mittels der Vorgabe eines Benutzers über Drücken der Tasten eines Betätigungsbildschirmes 600 steuern kann. Die hier beschriebene digitale Steuerungseinheit 60 umfasst nämlich einen Betätigungsbildschirm 600, mittels dessen über eine händische Betätigung des Benutzers elektronisch, vorzugsweise nur elektronisch, der Konzentrationsregler und/oder der Flussmengenregler steuer- und/oder regelbar ist. In einem solchen Fall kann daher der Betätigungsbildschirm 600 auf einer weiteren Montagefläche angeordnet sein, wobei diese weitere Montagefläche von der Montagefläche 42 verschieden ist. Zudem fällt der Betätigungsbildschirm 600 hierbei nicht unter ein Regelelement, welches auf der Montagefläche angeordnet ist. Dies kann heißen, dass in dem Beispiel der Figur 2B die Montagefläche eine solche Fläche ist, welche den Betätigungsbildschirm 600 ausschließt und dann zumindest teilweise umrandet. Dies kann heißen, dass über elektronische Signale, welche von dem Betätigungsbildschirm an eine Stelleinheit gesendet werden, diese Stelleinheit dann, beispielweise mechanisch, den Konzentrationsregler und den Flussmengenregler betätigt.

Insbesondere umfasst nämlich der Betätigungsbildschirm 600 zumindest ein Einstellfeld 601, auf dem der Benutzer händisch eine Konzentrationsänderung des Sauerstoffgases 11 und/oder des Lachgases 12 in vorgebbaren Änderungsschritten einstellen kann, wobei die Konzentrationsänderung in dem vorliegenden Ausführungsbeispiel nur durch die Betätigung des Einstellfeldes 601 einstellbar ist. Erkennbar ist jedoch jedenfalls wiederum der O₂-Notfallknopf 4, welcher abgesehen von einem Ein- und Ausschaltknopf der einzige Knopf innerhalb der Montagefläche 42 des Betätigungsfeldes 600 ist, so dass auch bei dem digitalen Lachgasmischer 100 es erfindungsgemäß gewährleistet ist, dass in einem Notfall der behandelnde Arzt besonders schnell den Patienten mit Sauerstoff fluten kann.

Die Erfindung ist nicht anhand der Ausführungsbeispiele beschränkt, vielmehr umfasst die Erfindung jedes neue Merkmal sowie jede Kombination von Merkmalen, was auch insbesondere jede Kombination der Patentansprüche beinhaltet, auch wenn dieses Merkmal oder diese Kombination selbst nicht explizit in den Patentansprüchen oder den Ausführungsbeispielen angegeben ist.

### Bezugszeichenliste

- 1: Mischkammer
- 1A: Mischkammergehäuse
- 2: Konzentrationsregler
- 3: Flussmengenregler/Flow-Regler
- 4: O₂-Notfallknop
- 6: Mischersteuerungseinheit
- 11: Sauerstoffgas
- 12: Lachgas
- 13: Lachgasgemisch
- 14: Vorkammer
- 15: Hauptkammer
- 15A: Halterung
- 15B: weitere Halterung
- 16: Raumluftventil
- 41: Betätigungsrichtung
- 42: Montagefläche
- 60: Steuerungseinheit
- 100: Lachgasmischer
- 110: Sauerstoffgasanschluss
- 110A, 120A: Gasleitungen für die beiden zu mischenden Gase
- 110AA, 120AA: Flowmeter
- 120: Lachgasanschluss
- 150: Gasbeutel
- 600: Betätigungsbildschirm
- 601: Einstellfeld

## Patentansprüche

1. Lachgasmischer (100) zur Erzeugung eines Lachgasgemisches, umfassend:
- zumindest eine Mischkammer (1), innerhalb derer ein Sauerstoffgas (11) und ein Lachgas (12) in jeweils vorgebbaren Volumina und/oder Molanteilen miteinander zu dem Lachgasgemisch (13) vermischt werden, wobei
- die Mischkammer (1) zumindest einen Lachgasanschluss (120) und zumindest einen Sauerstoffgasanschluss (110) aufweist, so dass über entsprechend an diesen Anschlüssen montierte Gasleitungen (110A, 120A) sowohl das Sauerstoff- als auch das Lachgas in die Mischkammer (1) eingeleitet wird,
- zumindest einen Konzentrationsregler (2), welcher dazu eingerichtet und dafür vorgesehen ist, ein Mischungsverhältnis zwischen dem in die Mischkammer (1) eingeleiteten Sauerstoffgas (11) und/oder dem in die Mischkammer (1) eingeleiteten Lachgas (12) in dem Lachgasgemisch vorgebbar einzustellen,
- zumindest einen Flussmengenregler (3), mittels dessen ein Volumenstrom der beiden Gase (11, 12) jeweils separat und/oder gemeinsam eingestellt werden kann,
**dadurch gekennzeichnet, dass**
zur Notflutung der Mischkammer (1) mit dem Sauerstoffgas (11) und/oder mit Umgebungsluft an der Mischkammer (1) zumindest ein O₂-Notfallknopf (4) angebracht ist, wobei ein Ein- und Ausschaltknopf des Lachgasmischers verschieden von einem Regelelement ist, und der O₂-Notfallknop (4) in einer Betätigungsrichtung (41) eindrückbar ist, wobei eine ebene Montagefläche (42) des O₂-Notfallknopfes (4), zu welcher die Betätigungsrichtung (41) an zumindest einem Punkt in der Montagefläche (42) senkrecht verläuft, frei von weiteren Regelelementen ist, und sich daher in der gesamten Nachbarschaft entlang und in der Ebene der Montagefläche kein weiteres Regelelement befindet, und wobei
- die Montagefläche (42) eine Außenfläche eines Mischkammergehäuses ist, an welcher der O₂-Notfallknopf angeordnet ist, oder
- die Montageflache eine Außenfläche einer Steuerungseinheit des Lachgasmischers ist, wobei eine solche Steuerungseinheit dann entfernt von dem Mischkammergehäuse angeordnet ist.

2. Lachgasmischer (100) nach Anspruch 1, **dadurch gekennzeichnet,**
**dass**
die Mischkammer (1) eine Vorkammer (14) sowie eine Hauptkammer (15) umfasst, wobei nur die Vorkammer (14) die beiden Gasanschlüsse (110, 120) umfasst und die Hauptkammer (15) über zumindest eine Fluidverbindung (130) mit der Vorkammer (14) verbunden ist, wobei ein Raumluftventil (16) anstatt an einer Halterung für die Hauptkammer befestigt zu sein abseits dieser Halterung angeordnet ist.

3. Lachgasmischer (100) nach Anspruch 2,
**dadurch gekennzeichnet, dass**
zumindest der O₂-Notfallknopf (4) an der Vorkammer (14) angeordnet sind.

4. Lachgasmischer (100) nach zumindest einem der Ansprüche 2 bis 3,
**gekennzeichnet durch**
zumindest eine Mischersteuerungseinheit (6), wobei die Mischersteuerungseinheit (6) zumindest eine digitale Steuerungseinheit (60) umfasst, wobei die Steuerungseinheit (60) derart ausgestaltet ist, dass diese den Konzentrationsregler (2) und/oder den Flussmengenregler (3) mittels der Vorgabe eines Benutzers steuert.

5. Lachgasmischer (100) nach dem vorhergehenden Anspruch,
**dadurch gekennzeichnet, dass**
die digitale Steuerungseinheit (60) zumindest einen Betätigungsbildschirm (600), insbesondere einen Touchscreen, umfasst, mittels dessen über eine händische Betätigung des Benutzers elektronisch der Konzentrationsregler (2) und/oder der Flussmengenregler (3) betätigbar sind, wobei
der Betätigungsbildschirm (600) zumindest ein Einstellfeld (601) umfasst, auf dem der Benutzer händisch eine Konzentrationsänderung des Sauerstoffgases (11) und/oder des Lachgases (12) in vorgebbaren Änderungsschritten einstellen kann, und wobei die Konzentrationsänderung durch die Betätigung dieses Einstellfeldes (601) einstellbar ist.

6. Lachgasmischer (100) nach zumindest einem der vorhergehenden Ansprüche,
**dadurch gekennzeichnet, dass**
der Lachgasmischer neben dem O₂-Notfallknopf (4) zudem einen Lachgasausleitknopf umfasst, über den eine Ausleitung des Lachgases aus der Mischkammer (1) sofort über die Dauer eines von einem Benutzer individuell einstellbaren Zeitraums unternehmbar und/oder über den Lachgasausleitknopf eine Zufuhr von Lachgas in die Mischkammer (1) für die Dauer dieses Zeitraums sofort unterbrechbar ist, und so nur noch Sauerstoff in die Mischkammer (1) zugeführt wird.

7. Lachgasmischer (100) nach dem vorhergehenden Anspruch,
**dadurch gekennzeichnet, dass**
der Lachgasausleitknopf ein Zeitsteuerelement umfasst oder von einem solchen gesteuert wird, sodass sich nach Ablauf einer an dem Zeitsteuerelement eingestellten Zeitdauer der Lachgasausleitknopf selbsttätig wieder in eine Nullstellung zurückstellt.

8. Lachgasmischer (100) nach zumindest einem der vorhergehenden Ansprüche,
**dadurch gekennzeichnet, dass**
an oder in zumindest einer der Gasleitungen und/oder unmittelbar an der Mischkammer (1) zumindest ein Flussmengensensor zur Messung einer Fließgeschwindigkeit und/oder eines Volumenstroms des jeweils in den Gasleitungen geleiteten Gases und/oder zur Messung eines Vakuumdurchflusses einer Absaugung angeordnet ist.

9. Lachgasmischer (100) nach dem vorhergehenden Anspruch,
**dadurch gekennzeichnet, dass**
dieser Flussmengensensor die Fließgeschwindigkeit und/oder den Volumenstrom des jeweiligen Gases, vorzugsweise ausschließlich, basierend auf einer Wärmetransportmessung des Gases ermittelt.

10. Lachgasmischer (100) nach einem der vorhergehenden Ansprüche 5 bis 9,
**dadurch gekennzeichnet, dass**
die Steuerungseinheit (60) dazu eingerichtet und dafür vorgesehen ist, dass während des Betriebs auf dem Behandlungsbildschirm eine Menüführung angezeigt wird, wobei über die Menüführung Behandlungsparameter, insbesondere Mengen- und/oder Flussangaben hinsichtlich des zugeführten Sauerstoff und/oder des Lachgases, individuell angezeigt und/oder gespeichert werden.

11. Lachgasmischer (100) nach dem vorhergehenden Anspruch,
**dadurch gekennzeichnet, dass**
die Steuerungseinheit (60) dazu eingerichtet und dafür vorgesehen ist, dass nach dem Anwählen auf dem Betätigungsbildschirm die zuletzt behandelten Patienten angezeigt werden, wobei dies entweder mittels eines anonymisierten Kodierungssystems geschieht oder auch mit dem konkreten Namen bzw. der entsprechenden Patienten-ID geschieht.

12. Lachgasmischer (100) nach zumindest einem der vorhergehenden Ansprüche,
**dadurch gekennzeichnet, dass**
dieser Gasventile zur Steuerung und/oder Durchleitung des Gasflusses des Sauerstoffs und/oder des Lachgases umfasst, wobei die Gasventile als Steuerungsmittel, anstatt Gummimembranen, jeweils einen Steuerkolben vorsehen.

13. Lachgasmischer (100) nach zumindest einem der vorhergehenden Ansprüche,
**dadurch gekennzeichnet, dass**
dieser ein akustisches Warnmittel umfasst, wobei ein solches Warnmittel dazu eingerichtet und dafür vorgesehen ist den Fluss eines oder beider Gase zu unterbrechen, wobei dann unmittelbar nach der Unterbrechung des jeweiligen Gasflusses ein insbesondere akustisches Warnsignal ertönt.

14. Lachgasmischer (100) nach zumindest einem der vorhergehenden Ansprüche 2 bis 13,
**dadurch gekennzeichnet, dass**
eine Flächennormale einer Ventilöffnung des Raumluftventils (16) nach unten und im Wesentlichen parallel zur Hauptersteckungsrichtung der Hauptkammer (15) verläuft.

15. Lachgasmischer (100) nach zumindest einem der vorhergehenden Ansprüche 2 bis 14,
**dadurch gekennzeichnet, dass**
dieser zumindest einen USB-Port und/oder einen SD-Kartenanschluss umfasst, sodass für Serviceeinsätze eine vorgegebene Anzahl von Fehlern, vorzugsweise alle Fehler, ausführbar sind, insbesondere wobei
- die Fehler in einem Administratormenü immer nachzulesen und zu exportieren sind, und/oder
- der Behandler mit einem USB Stick und/oder einem SB-Port Protokolle z.B. mit einer Patientennummer speichern kann, und/oder
- der Behandler die Behandlungsdauer, die Konzentration etc. dokumentieren kann.

16. Lachgasmischer (100) zumindest einem der vorhergehenden Ansprüche 2 bis 15,
**dadurch gekennzeichnet, dass**
an der Mischkammer (1) zumindest ein Drucksensor angeordnet ist, welcher zumindest einen der Gasdrücke misst, wobei über eine mit dem Drucksensor innerhalb eines Regelkreises verbauten Regeleinheit die einzelnen Gasdrücke während der Behandlung oder auch vorher ablesbar und/oder einstellbar sind.

17. Lachgasmischer (100) zumindest einem der vorhergehenden Ansprüche,
**dadurch gekennzeichnet, dass**
dieser einen Fernbedienungsanschluss für eine Remotecontrol umfasst, wobei somit die Steuerungseinheit (60) einen Kabel- und/oder IR- und/oder Bluetoothanschluss umfasst und/oder über Funk betreibbar ist.

18. Lachgasmischer (100) zumindest einem der vorhergehenden Ansprüche 2 bis 17,
**dadurch gekennzeichnet, dass**
dieser eine Sprachsteuerungsvorrichtung umfasst, wobei diese dazu eingerichtet und dafür vorgesehen ist, vorzugsweise alle, Funktionen über eine akustische Detektion einer Behandlerstimme zu steuern.

19. Lachgasmischer (100) nach zumindest einem der vorhergehenden Ansprüche,
**dadurch gekennzeichnet, dass**
der Lachgasmischer frei von einem An-und Ausschaltknopf ist.
